# EUROPEAN PATENT APPLICATION

(11) **EP 2 339 008 A1**
(43) Date of publication of application: **29.06.2011**
(21) Application number: 09180669.5
(22) Date of filing: 23.12.2009
(51) Int. Cl.: C12N 15/82, A01H 5/00

(54) **Basal transcription factors silencing to induce recoverable male sterility in plants**

(71) Applicant: Universita' Degli Studi Di Milano, 20122 Milano (IT); CRA Consiglio per la Ricerca e la sperimentazione in Agricoltura, 00184 Roma (IT)
(72) Inventor: Colombo, Lucia, I-20122 Milano (IT); Kater, Martin, I-20122 Milano (IT); Kooiker, Maarten Christiaan, I-20133 Milano (IT); Lindner, Matias, I-20133 Milano (IT); Rotino, Giuseppe Leonardo, I-00184 Roma (IT); Toppino, Laura, I-00184 Roma (IT)
(74) Representative: Long, Giorgio

(57) **Abstract**

The present invention concerns the inducting of recoverable male sterility in plants through the introduction into the plant genome of a genic construct comprising a blocking construct, which blocks the activity of a gene involved into the pollen formation and a rescue construct, which is able to restore fertility under suitable conditions.

SEQUENCE LISTING

<110>  Università degli Studi di Milano
       CRA Consiglio per la Ricerca e sperimentazione in
       Agricoltura

<120>  Basal transcription factors silencing to induce recoverable male
       sterility in plants

<130>  E0075170/VCR

<160>  35

<170>  PatentIn version 3.5

<210>  1
<211>  22
<212>  DNA
<213>  Artificial Sequence

<220>
<223>  primer

<400>  1
tatactccca ctattcctga tg                                              22


<210>  2
<211>  21
<212>  DNA
<213>  Artificial Sequence

<220>
<223>  primer

<400>  2
tcattcctct cttgaagcag g                                               21


<210>  3
<211>  20
<212>  DNA
<213>  Artificial Sequence

<220>
<223>  primer

<400>  3
atgaacaact cttctgcagg                                                 20


<210>  4
<211>  20
<212>  DNA
<213>  Artificial Sequence

<220>
<223>  primer

<400>  4
acttcccctt cactgatgtg                                                 20


<210>  5
<211>  40
<212>  DNA
<213>  Artificial Sequence

<220>

<223>  oligo

<400>  5
gaattaatcg aacgctagga cattctctct tttgtattcc                              40


<210>  6
<211>  40
<212>  DNA
<213>  Artificial Sequence

<220>
<223>  oligo

<400>  6
gaatgtccta gcgttcgatt aattcaaaga gaatcaatga                              40


<210>  7
<211>  40
<212>  DNA
<213>  Artificial Sequence

<220>
<223>  oligo

<400>  7
gaatatccta gcgttggatt aattcacagg tcgtgatatg                              40


<210>  8
<211>  40
<212>  DNA
<213>  Artificial Sequence

<220>
<223>  oligo

<400>  8
gaattaatcc aacgctagga tattctacat atatattcct                              40


<210>  9
<211>  40
<212>  DNA
<213>  Artificial Sequence

<220>
<223>  oligo

<400>  9
gattgtggca agtcctttct acttctctct tttgtattcc                              40


<210>  10
<211>  40
<212>  DNA
<213>  Artificial Sequence

<220>
<223>  oligo

<400>  10
gaagtagaaa ggacttgcca caatcaaaga gaatcaatga                              40


<210>  11
<211>  40
<212>  DNA

<213>  Artificial Sequence

<220>
<223>  oligo

<400>  11
gaagcagaaa ggactagcca cattcacagg tcgtgatatg                              40


<210>  12
<211>  40
<212>  DNA
<213>  Artificial Sequence

<220>
<223>  oligo

<400>  12
gaatgtggct agtcctttct gcttctacat atatattcct                              40


<210>  13
<211>  29
<212>  DNA
<213>  Artificial Sequence

<220>
<223>  oligo

<400>  13
caccctgcaa ggcgattaag ttgggtaac                                          29


<210>  14
<211>  28
<212>  DNA
<213>  Artificial Sequence

<220>
<223>  oligo

<400>  14
gcggataaca atttcacaca ggaaacag                                           28


<210>  15
<211>  32
<212>  DNA
<213>  Artificial Sequence

<220>
<223>  oligo

<400>  15
gtcgactcta gactttttgg ttagcgaatg ca                                     32


<210>  16
<211>  35
<212>  DNA
<213>  Artificial Sequence

<220>
<223>  oligo

<400>  16
ccatggtcga ctagctaatt tctttaagta aaaac                                   35

<210>  17
<211>  31
<212>  DNA
<213>  Artificial Sequence

<220>
<223>  oligo

<400>  17
gtcgactcta gagccatggt taatcactaa g                                      31


<210>  18
<211>  29
<212>  DNA
<213>  Artificial Sequence

<220>
<223>  oligo

<400>  18
ccatggtcga cggatcgatg ttggttatc                                         29


<210>  19
<211>  23
<212>  DNA
<213>  Artificial Sequence

<220>
<223>  primer

<400>  19
caccggttgg tggcagttgc tac                                               23


<210>  20
<211>  23
<212>  DNA
<213>  Artificial Sequence

<220>
<223>  primer

<400>  20
caccggagat gatagcaatc cac                                               23


<210>  21
<211>  20
<212>  DNA
<213>  Artificial Sequence

<220>
<223>  oligo

<400>  21
tgctcctgcc gagaaagtat                                                   20


<210>  22
<211>  23
<212>  DNA
<213>  Artificial Sequence

<220>
<223>  oligo

<400>  22

agaactcgtc aagaaggcga tag 23

<210> 23
<211> 21
<212> DNA
<213> Artificial Sequence

<220>
<223> oligo

<400> 23
tgtcaaggta aggcgtttgt c 21

<210> 24
<211> 23
<212> DNA
<213> Artificial Sequence

<220>
<223> oligo

<400> 24
ggagatgata gcaatccact tcc 23

<210> 25
<211> 22
<212> DNA
<213> Artificial Sequence

<220>
<223> oligo

<400> 25
aagccttccg agcttgtttc ag 22

<210> 26
<211> 315
<212> DNA
<213> Solanum melongena

<400> 26
atgaattggt gtgagcatta cttgggtaaa agtggatttc aatgtcctga cgattcagat 60

taataaggtt ggtggcagtt gctacacaga agtttatatg cagatgttgc cactgatgct 120

ctccaacatt gcaaggcaag gcagtcaact attgtcaagg ataaaagaga taagcagcaa 180

aaggacaaac gccttacctt gacaatggat gatctttcaa aatctctacg agagtatggt 240

gtaaacgtca aacatcaaga ctactttgct gatagcccat ctgctggatt agatcctgct 300

tcaagagagg aatga 315

<210> 27
<211> 396
<212> DNA
<213> Solanum melongena

<400> 27
atgaacaaca cttctgcagg gccatcgtca aaagctagag taggagcatc acaaccttca 60

gaaagttcac ttaaaaggaa acgtggcatg tttcagaaag acttgcaaca tatgatgtat 120

ggttttggag atgatagcaa tccacttccg gaaactgtgg cacttgtgga ggacatcgtg 180

gtggattatg tcactgatat ggtacacaaa gctcaggatg ttgcaagcaa aagggggaag 240

cttttgacag aggacttctt attcttaatt agaaaggact tgccaaaact taaccggtgt 300

acagaactgt tgtccatgaa tgaggaactg aaacaagctc ggaaggcttt tgaggtcgat 360

gaagagaaat tcccatcaca tcagtgaagg ggaagt 396

<210> 28
<211> 311
<212> DNA
<213> Lycopersicon esculentum

<400> 28
atgaattggt ggagcattac ttgggtaaaa gtggatttca atgtcctgat gttcgattaa 60

taaggttggt ggcagttgct actcagaagt ttattgcaga tgtggctact gatgctctcc 120

aacattgcaa ggcaaggcag tcaactattg tcaaggacaa gagagataag cagcaaaagg 180

acaaacgcct taccttgaca atggatgatc tttcaaaatc tctacgagag tatggtgtta 240

acgtcaaaca tcaagactac tttgctgata gcccgtctgc cgggttagat cctgcttcaa 300

gagaggaatg a 311

<210> 29
<211> 396
<212> DNA
<213> Lycopersicon esculentum

<400> 29
atgaacaact cttctgcagg gccatcctca aaagctagag taggagcatc acaaccttca 60

gaaagttcac ttaagaggaa acgtggcatg tttcagaaag atttgcaaca catgatgtat 120

ggttttggag atgatagcaa tccacttcca gaaactgtgt cacttgttga agacatcgtg 180

gtggattatg tcactgatat ggtgcacaaa gctcaggata ttgcaaccaa aagggggaag 240

cttttgaccg aggactttct gttcttaatt agaaaggact tgccgaaact taaccggtgt 300

acagaactgt tgtccatgaa tgaggagctg aaacaagctc ggaaggcttt cgaggtcgat 360

gaagagaaat tggcttcaca tcagtgaagg ggaagt 396

<210> 30
<211> 862
<212> DNA
<213> Nicotiana tabacum

<400> 30
ctttttggtt agcgaatgca attaatttag acattgtgtt atgttccagt taaccgcttc 60

cctgcacttc tttcaatcta tctctcgata gaaaattgtg atactttgcg acttctatca 120

gaggactttt tgttttccat gtaacaatct gtcattttcg atggggagat ttgcacaaat 180

aggctattta tgtgtcccaa tttaaatttt aaccccatgt cgatcagaac ttagccacga 240

gcaccagaag tttgatggat atgtgacttt gtcactatcc ggtttactaa tcaagagcta 300

tttttattca aaattggata tctagctaag tataactgga taatttgcat taacagattg 360

aatatagtgc caaacaagaa gggacaattg acttgtcact ttatgaaaga tgattcaaac 420

atgatttttt atgtactaat atatacatcc tactcgaatt aaagcgacat aggctcgaag 480

tatgcacatt tagcaatgta aattaaatca gtttttgaat caagctaaaa gcagacttgc 540

ataaggtggg tggctggact agaataaaca tcttctctag cacagcttca taatgtaatt 600

tccataactg aaatcagggt gagacaaaat tttggtactt tttcctcaca ctaagtccat 660

gtttgcaaca aattaataca tgaaacctta atgttaccct cagattagcc tgctactccc 720

cattttcctc gaaatgctcc aacaaaagtt agttttgcaa gttgttgtgt atgtcttgtg 780

ctctatatat gcccttgtgg tgcaagtgta acagtacaac atcatcactc aaatcaaagt 840

ttttacttaa agaaattagc ta 862

<210> 31
<211> 648
<212> DNA
<213> Nicotiana tabacum

<400> 31
gccatggtta atcactaaga aatttagggt tggtactcta agatgaatta tgaaggtagc 60

tatagtgggg ggttttatag ggttgtaaag gaagagatat tcataagtat taggagagtt 120

attaacttta tcgaagatat gaatattgat ttatgtcaat cataaatact gaagtttgca 180

tacaaagaga attcttaaaa tgcatgcaat ttgaagtaga taaagagtga acaagatttc 240

cctatatcta ttcaaaatct tatatgaaaa agaaaataaa taagaggggt cctcgtctga 300

tcaattagaa gcaaaagatc tcttgattac attaactttt tttaaatgag gaattgtttt 360

gtaatcaact taataaacta tttaatttct gctagctggt attaacttgt atagtccatt 420

tatatgaata ctagcttttt tgaccggttt ggtttgtatc aaattctttt gcccgattta 480

ttccctactt ctattaactt aatagatttt attaatcacg ttgtttgatt atgatatgaa 540

aagcatttta acacttttgg ggtttttttc gagaacaaag tgatttattt aacttagtgg 600

tgagacgtcc aaagtggttg gtccttagtt gataaccaac atcgatcc 648

<210> 32
<211> 466
<212> DNA
<213> Artificial Sequence

<220>
<223> amiR-SmTAF10

<400> 32
tcgaggtcga cggtatcgat aagcttgata tgaattcctg cagccccaaa cacacgctcg 60

gacgcatatt acacatgttc atacacttaa tactcgctgt tttgaattga tgttttagga 120

atatatatgt agaatatcct agcgttggat taattcacag gtcgtgatat gattaattag 180

cttccgactc attcatccaa ataccgagtc gccaaaattc aaactagact cgttaaatga 240

atgaatgatg cggtagacaa attggatcat tgattctctt tgaattaatc gaacgctagg 300

acattctctc ttttgtattc caattttctt gattaatctt tcctgcacaa aaacatgctt 360

gatccactaa gtgacatata tgctgccttc gtatatatag ttctggtaaa attaacattt 420

tgggtttatc tttatttaag gcatcgccat ggggggatcc ctagtt 466

<210> 33
<211> 466
<212> DNA
<213> Artificial Sequence

<220>
<223> amiR-SmTAF13

<400> 33
tcgaggtcga cggtatcgat aagcttgata tgaattcctg cagccccaaa cacacgctcg 60

gacgcatatt acacatgttc atacacttaa tactcgctgt tttgaattga tgttttagga 120

atatatatgt agaagcagaa aggactagcc acattcacag gtcgtgatat gattaattag 180

cttccgactc attcatccaa ataccgagtc gccaaaattc aaactagact cgttaaatga 240

atgaatgatg cggtagacaa attggatcat tgattctctt tgattgtggc aagtcctttc 300

tacttctctc ttttgtattc caattttctt gattaatctt tcctgcacaa aaacatgctt 360

gatccactaa gtgacatata tgctgccttc gtatatatag ttctggtaaa attaacattt 420

tgggtttatc tttatttaag gcatcgccat ggggggatcc ctagtt 466

<210> 34
<211> 21
<212> RNA
<213> Artificial Sequence

<220>
<223> amiRNA-TAF13

<400> 34
uuguggcaag uccuuucuac u 21

<210> 35
<211> 21
<212> RNA
<213> Artificial Sequence

<220>
<223> amiRNA-TAF10

<400> 35
auuaaucgaa cgcuaggaca u 21

## Description

### DESCRIPTION OF THE INVENTION

The present invention finds application in the field of agriculture and, in particular, for the preparation of recoverably male sterile plants.

### BACKGROUND

Since decades male sterility, i.e. the inability of a plant to produce fertile pollen, has been considered an useful tool to prevent unwanted self-pollination during the breeding process and the hybrid seed production (Kaul, 1988), which plays an essential role in agriculture. Essentially, a F1 hybrid plant is obtained through a breeding process, in which so-called inbred lines are crossed to create seeds with greater yield potential than their parents due to an effect called heterosis or hybrid vigor. Said process allows plant breeders to enhance biological characteristics or traits more predictably and more quickly than other methods. At the same time, it also protects the intellectual property by keeping knowledge of their hybrid varieties. In fact, the enhanced vigor of hybrid varieties is not transmitted to their offspring. Most crops show hybrid vigor, but the commercial production of hybrids is only feasible if a reliable and cost effective control of pollination is available.

However, for some crops that are not usually vegetatively propagated, an irreversible system is undesirable, because of the inability to propagate the male-sterile female parent line in a cost-effective way; therefore, strategies for making the male sterility trait reversible, yet tightly controllable, are needed

The development of an efficient system to induce male sterility in plants could also answer to the many raising concerns regarding GM crops cultivation, mainly because of environmental, economical and political risks. One of the mayor concerns, in fact, is the transgene flow from GM crops to wild relatives or traditional crop fields. For some crops, the prevention of pollen flow between target and non-target plants can be physically impossible, as it is mediated by environmental factors, such as wind, insects, and the proximity of sexually compatible weedy relatives. All these factors are beyond the control of the breeder and farmer, thus leading to the necessity of developing tightly controllable alternative strategies for the biological containment of the transgene. (Kuvshinov et al., 2001).

Among the strategies for biological containment, male-sterility provides an effective way for preventing gene flow from GM plants to the environment (Daniell,1998 and 2002), and also allows for the development of an effective system for hybrid seeds production. Several strategies aimed at creating male sterility have been described (Perez-Prat and van Lookeren Campagne,2002), of which some rely on natural cytoplasmic (CMS, with sugar beet and oilseed rape as examples), genetic or functional male sterility and restorer systems, and others on the use of genetic modification. The latter can be grouped into a number of broad classes including systems that rely on: 1) metabolic starvation; 2) anther or pollen-lethal mutations in endogenous genes; 3) two component in which the two halves of a cytotoxic compound are brought together in the anther after crossing. Engineered male sterility for F1 hybrid breeding has been obtained by using a one-component strategy in which a promoter is used to drive the expression of a single cytotoxic gene (RNase or Barnase) in pollen or tapetal cells. The technology is convenient for hybrid seed production, but does not allow transgene containment of male sterile hybrid seed. Another drawback of the one-component system is that it suffers from the inability to produce pure strands of homozygous male sterile plants, since transgenic plants carrying the promoter-Barnase like construct cannot be selfed; therefore these systems need implementation for the recovery of the block-of-function. In some cases this drawback may be overcome if an efficient method to obtain anther-derived doubled-haploid plants is available. Among the *Solanum* genera, different approaches for the obtainment of conditional male sterile plants have been reported in petunia (Napoli et al., 1990, Derksen,1999), tobacco (kiete et al, 1996. Fisher et al., 1997; Cho et al.,2001, Bayer et al., 2005) and tomato (Mariani et al., 1990 an 1992, Ribarits et al.,2007) most of them including a system for the recovery of pollen fertility, but all based on the introduction of foreign genes into the host genome. The introduction of foreign traits of DNA into a crop raised a strong opposition against the consumption of transgenic foods, aiming many efforts towards the developing of more acceptable engineering strategies like the insertion of native rather than foreign elements into the genome (Rommens C.M. 2004).

### SUMMARY OF THE INVENTION

The present invention discloses a method for the recoverable induction of male sterility in plants, which method overcomes the drawbacks of the methods already known in the art.

In particular, it is proposed a recoverable containment strategy that prevent gene transmission via pollen. The strategy for inducing male sterility is based on a tissue-specific artificial microRNA-mediated (Shwab et al., 2006)loss-of-function of two TBP Associated factors TAF10 and TAF13 (Lago et al., 2004), which are known to be expressed in anther tapetal and microsporal tissue and to play a key role during pollen development (Gao et al., 2005, Furumoto et al., 2005). The amiRNA target a specific sequence of the two TAF genes under the control of the pTA29 and pTM19 promoters, which are known to rule tissue-specific expression in the tapetum and microspore, respectively (Koltunow et al., 1990; Custers et al., 1997). The ability to complement potential male sterile plant to restore fertility is based on the inducible expression of a miRNA-insensitive form of the target TAF gene. In particular, the present method allows the recovery of the blocked function only upon an external stimulus, such as treatment or intervention. The so obtained male sterile plants will not spread the transgene in the environment.

On the other hand, the rescue of male fertility is desired to obtain seeds for commercial or breeding purposes.

### DESCRIPTION OF THE FIGURES

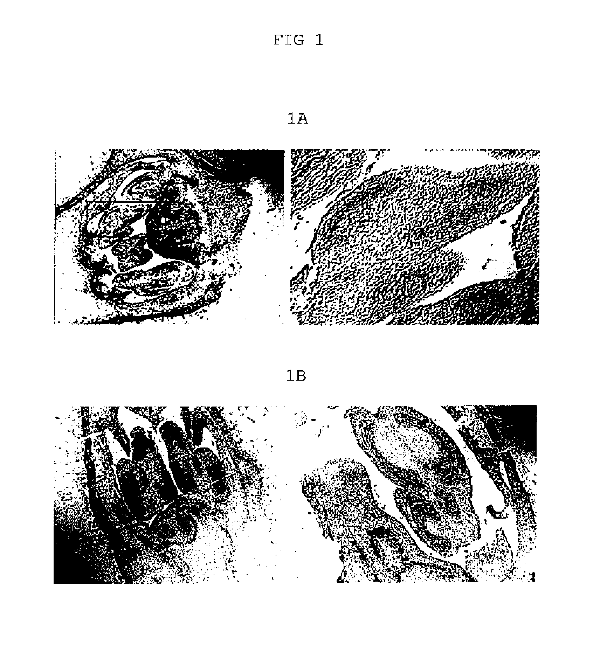

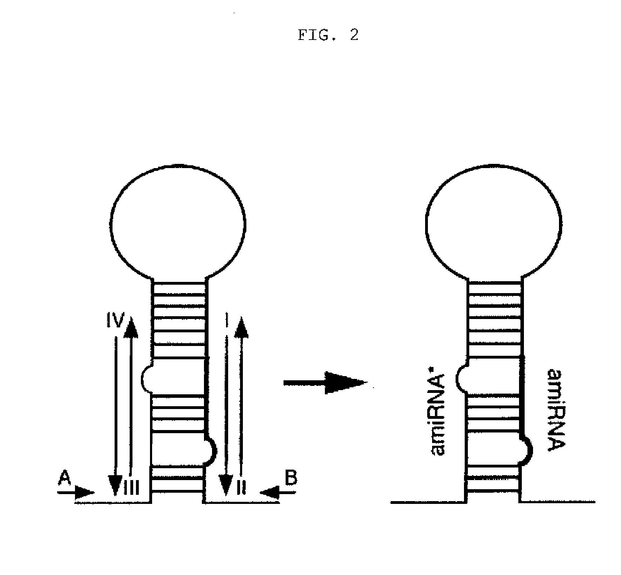

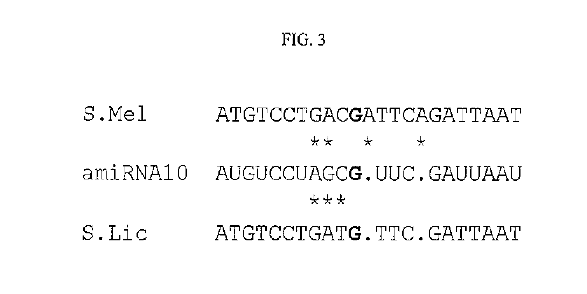

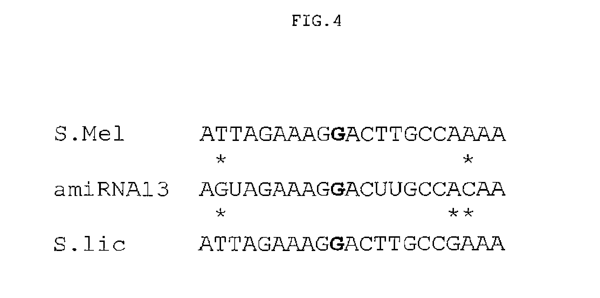

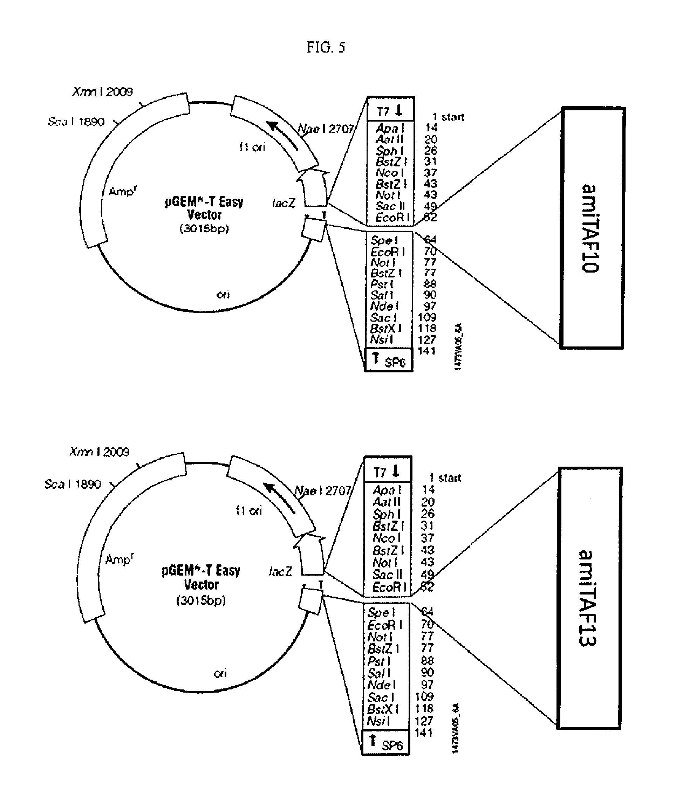

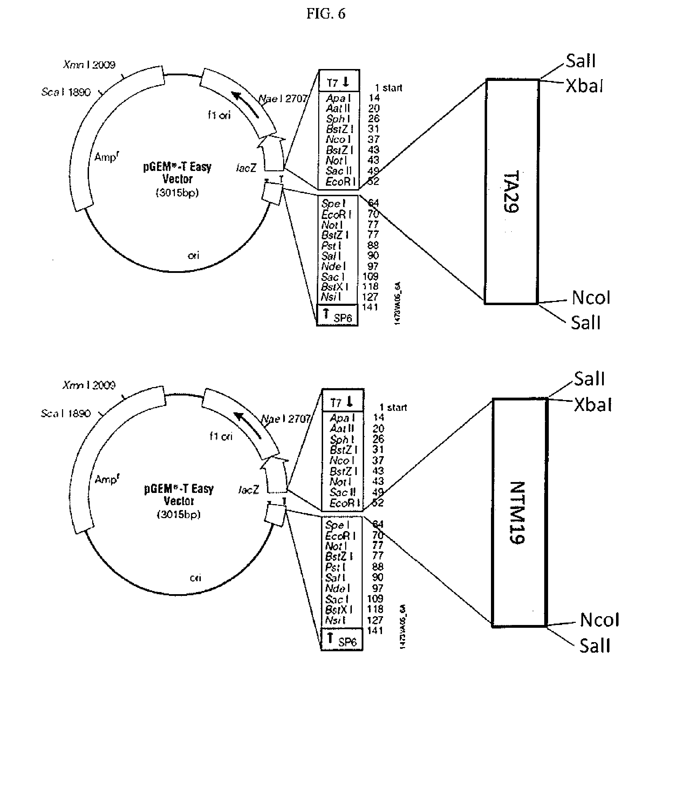

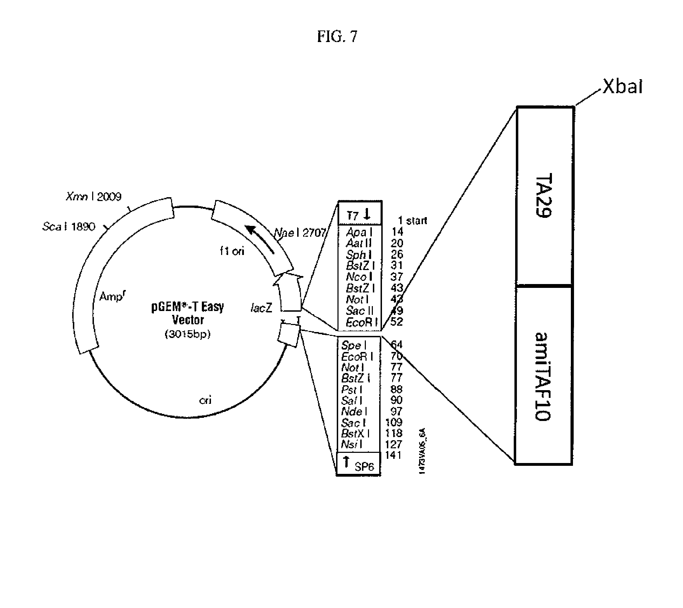

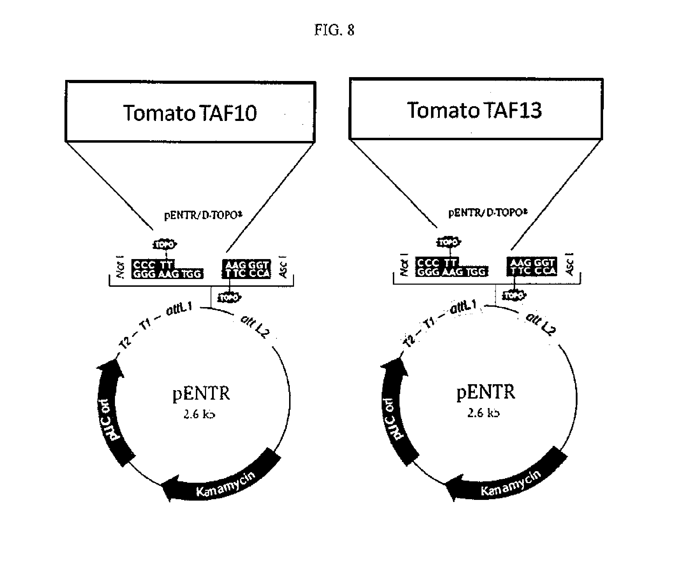

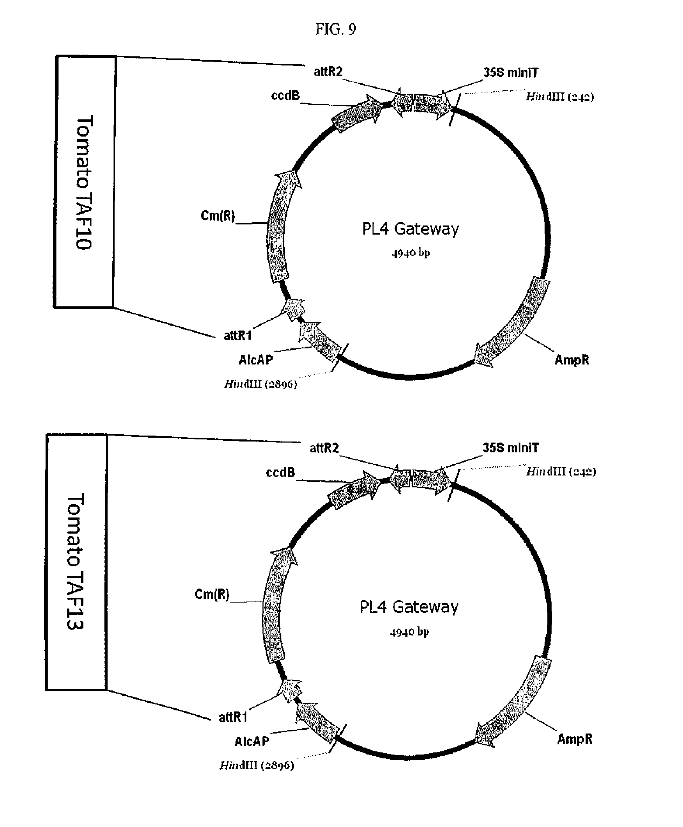

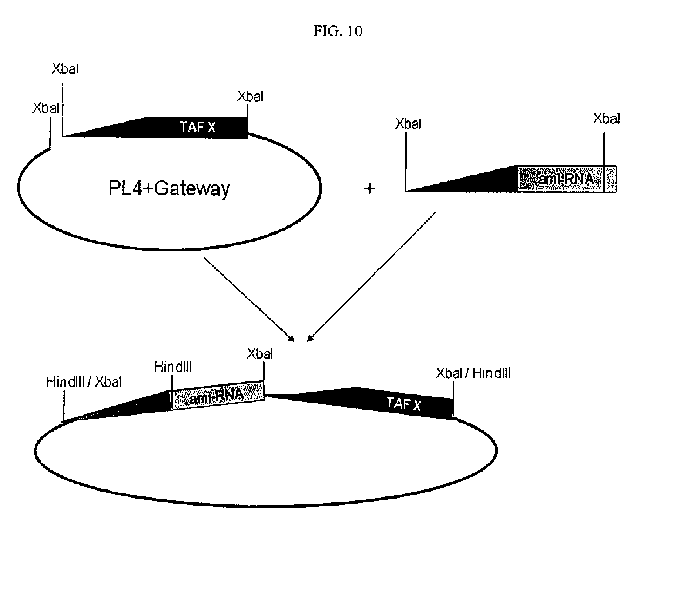

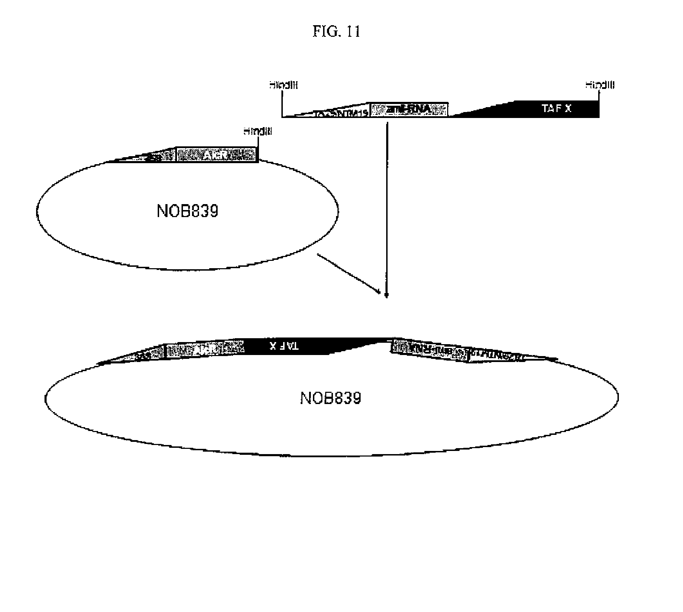

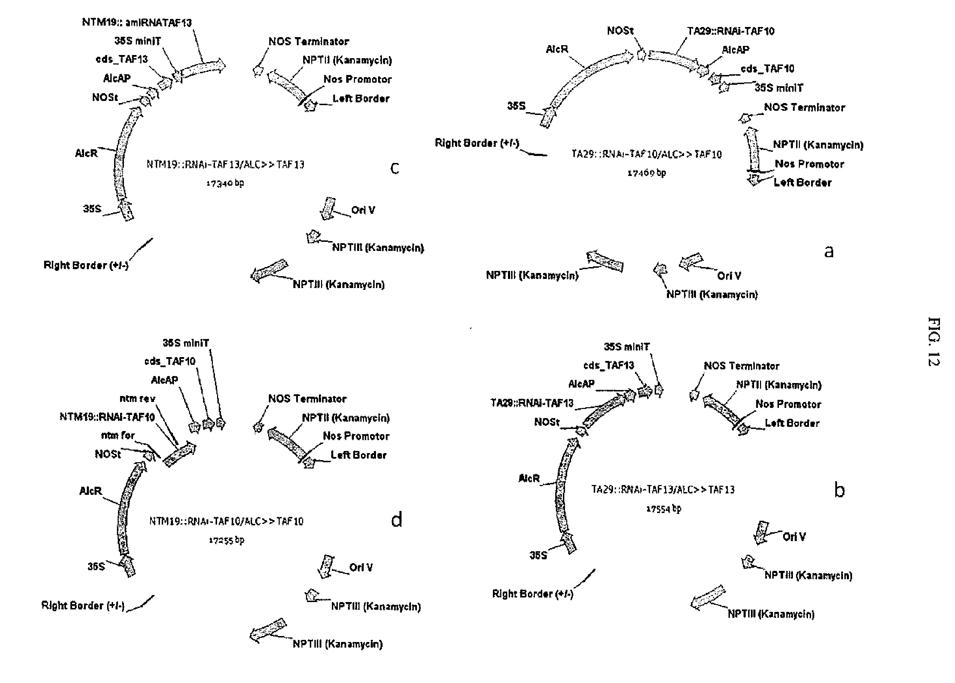

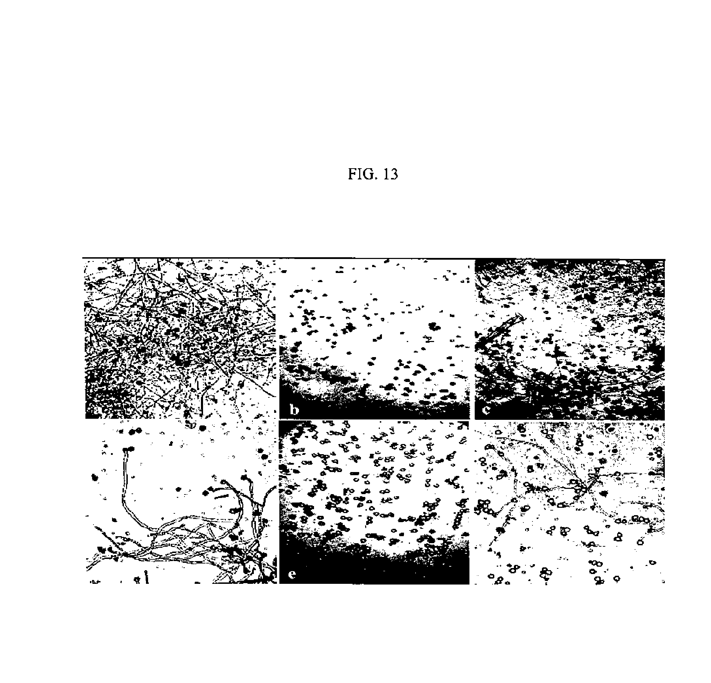

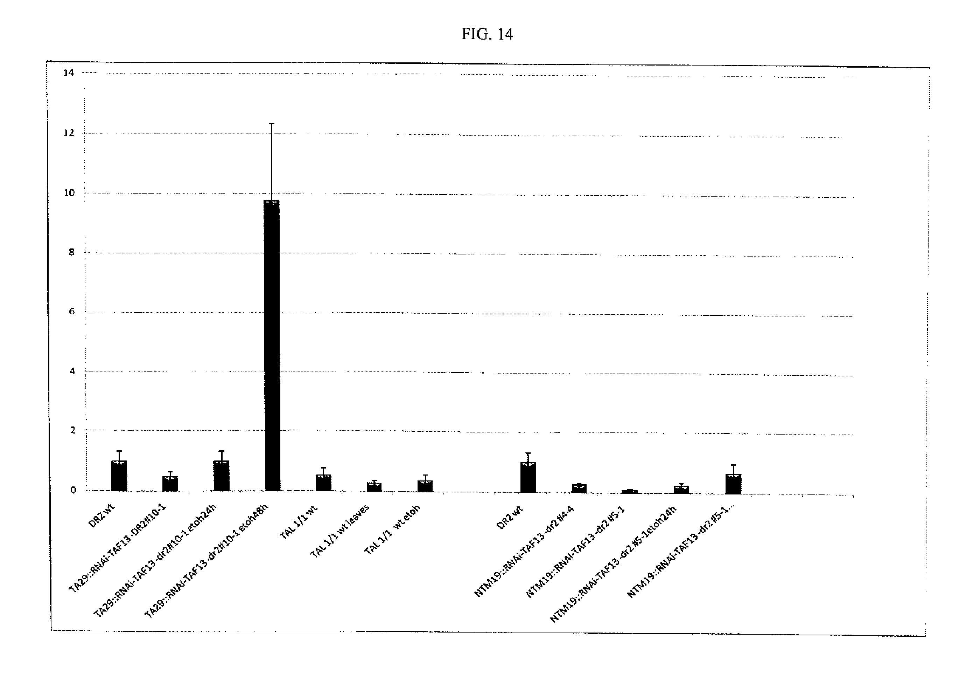

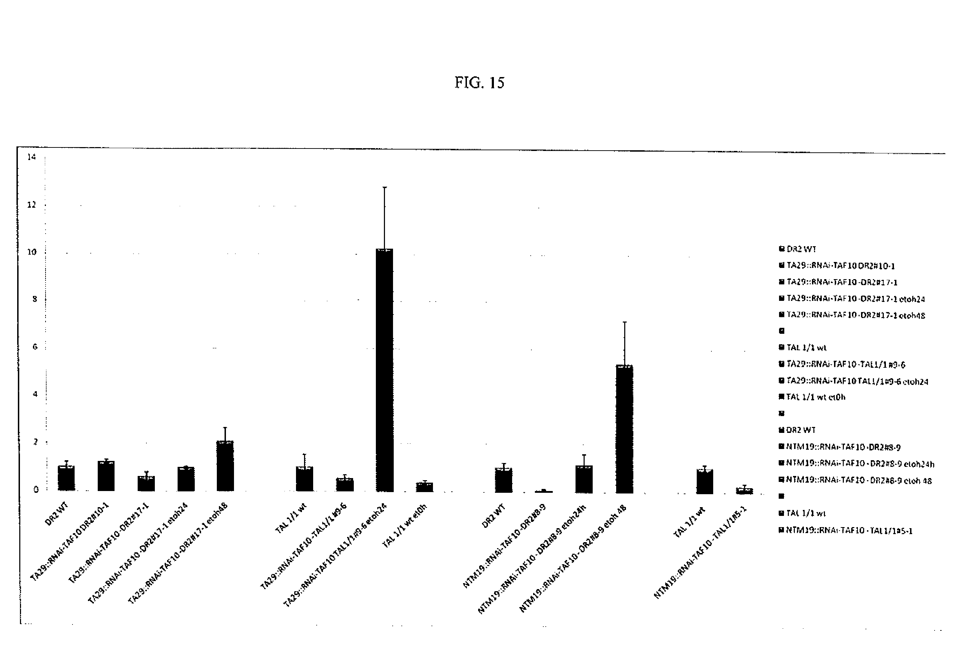

Figure 1A shows the *in situ* analysis for TAF10 expression in eggplant flowers, while Figure 1B shows the *in situ* analysis for TAF13 expression in eggplant flowers; Figure 2 shows a graphic representation of the alignment of the four primers to form the amiRNA precursor (Schwab et al., 2006); Figure 3 is a graphical representation of the mismatches existing between the TAF10 microRNA sequence and the correspondent target sequences in *S. melongena* and *S. lycopersicum;* Figure 4 is a graphical representation of the mismatches existing between the TAF13 microRNA sequence and the correspondent target sequences in *S*. *melongena* and *S*. *lycopersicum;* Figure 5 shows amiR-smTAF10 and amiR-smTAF13 cloned in pGEM T-EASY vector; Figure 6 shows the promoters pTA29 and pNTM19 cloned in pGEM-T EASY vectors; Figure 7 shows pTA29::amiR-smTAF10 cloned in pGEM-T easy vector; Figure 8 represents the tomato TAF10 and Tomato TAF13 cloned in TOPO vector; Figure 9 shows the Tomato TAF10 and Tomato TAF13 cloned under pALCA promoter in PL4 Vector through Gateway reaction; Figure 10 is a schematic representation for the obtainment of the complete construct pTA29 (pNTM19) : :amiR-smTAF; pALCA: : tomatoTAF into PL4 vector; Figure 11 is a schematic representation for the obtainment of the complete constructs p35S::ALCR; pTA29(pNTM19)::amiR-smTAF; pALCA::tomatoTAF; Figure 12 shows the binary vectors for agrobacterium-mediated transformation containing: a) p35S::ALCR; pTA29::amiR-smTAF10; pALCA::tomatoTAF10, b) p35S::ALCR; pTA29::amiR-smTAF13; pALCA::tomatoTAF13, c) p35S::ALCR; pNTM19::amiR-smTAF13; pALCA::tomatoTAF13, d) p35S::ALCR; pTM19::amiR-smTAF10; pALCA::tomatoTAF10; Figure 13 shows the Pollen germination ratio in PEG 800 in wild type and transformed plants, both without and after ethanol induction. a) TAL 1 / 1wt, b)pTA29::amiR-smTAF10 pALCA::tomatoTAF10, TAL1/1#9-6, c) pTA29::amiR-smTAF10, pALCA::tomatoTAF10 TAL1/1#9-6 + etoh48h, d):DR2 wt, e) pNTM19::amiR-smTAF13 pALCA::tomatoTAF13, DR2#5-1, f): pNTM19::amiR-smTAF13; pALCA::tomatoTAF13, DR2#5-1 + etoh48h; Figure 14 shows a real time analysis for the expression of TAF13 in wild type and transformed plants, both without and after ethanol induction; Figure 15 shows a real time analysis for the expression of TAF10 in wild type and transformed plants, both without and after ethanol induction.

### OBJECT OF THE INVENTION

In a first object of the present invention, there is provided a genic construct, which can be suitably used for imparting recoverable male sterility in plants, as per claims 1 to 21.

In particular, said genic construct comprises a blocking construct and a recovery construct.

More in particular, said blocking construct comprises an anther-specific promoter operably associated to a nucleotidic sequence capable of inactivating a target gene coding for a basal transcription factor involved in polled development, so that its expression may block the production of functional pollen and therefore cause male sterility in plant.

As for the rescue construct, it comprises an inducible promoter operably associated to a form of said gene involved in pollen development, which form is insensitive to such sequence capable of inactivating the target gene. In a further embodiment, it is disclosed a method for preparing a genic construct capable of inducing recoverable male sterility in plants, as per claims 22 to 25.

In a still further embodiment, the present invention discloses the preparation of recoverably male sterile plants, according to claims 26 to 30.

Still, there are disclosed a recombinant plant cell bearing the genic construct of the invention as well as a plant transformed with said genic construct and seeds obtained from said plant as per claim 31.

In a preferred embodiment of the invention, the modified cell, and accordingly the modified plant and seeds, is of

### Solanum melongena.

Further objects will be apparent from the following description and exemplified implementation of the invention.

### DETAILED DESCRIPTION OF THE INVENTION

### Definitions

Within the present invention, "artificial micro RNA" or "amiRNA" is used to indicate an artificial sequence, which is capable of recognizing and inactivating a target gene (also referred to as gene of interest).
"Target gene" refer to the gene the blocking construct of the invention is directed to thus inactivating it. In particular, the target gene is TAF10 or TAF13 genes.

In a preferred embodiment, said target TAF10 and TAF13 genes are from *Solanum melongena* (accordingly, referred to as smTAF10 and smTAF13, respectively). Thus, "amiRNA-TAF10" and "amiRNA-TAF13" refer to the artifical sequences specifically targeting the TAF10 and TAF13 genes, which are, as per the preferred embodiment of the invention, from *Solanum melongena* (amiRNA-smTAF10 and amiRNA-smTAF13, respectively).

"Construct" means an artificially constructed nucleotide/polynucleotides sequence or nucleic acid; "blocking construct" means a construct, which is able to bind to a specific target and to block its function, while "recovery/rescue/restoration construct" is a construct, which is able to restore the activity blocked by the blocking construct.

A "amiRNA-insensitive form of the gene" or "insensitive (form of the) gene" is a genetic sequence, which is capable of producing the same activity of the sequence targeted, but which is not blocked by the blocking construct.

A "blocking vector" is a vector, i.e. a nucleic acid molecule, such as a plasmid, a cosmid, a bacteriophage, etc, which is capable of replicating autonomously in a host cell and comprises the blocking construct, while a "rescue vector" is a nucleic acid molecule as above said, which conversely comprises the recovery construct.

With the term "two-component construct" is meant a vector comprising both the blocking and the recovery vector ligated thereto, which two-component construct is comprised within a "binary vector".

"Promoter" refers to a nucleic acid sequence that directs the transcription of an associated gene; the promoter may be specific for some particular tissues of a plants.

"Operably linked" or "operably associated" refers to the functional linkage existing between a first sequence, such as a promoter and a second sequence, wherein the first sequence, i.e. a promoter, initiates and mediates the transcription of the DNA corresponding to the second sequence. Generally, operably linked sequences are contiguous.

An "host cell" is a cell which is transformed with a vector containing the genic construct of the invention and which is able to transfer it into another cell.

For the purposes of the present invention, a preferred host cell is *Agrobacterium tumefaciens.*

A "transformed cell" is a cell comprising an artificial construct which has been introduced therein by suitable and well known techniques in the art; a plant comprising transformed cells is a "transformed plant".

A "male sterile plant" is a plant which is not able to produce functional pollen, while a "recoverably male sterile plant" is a plant wherein male fertility may be restored under suitable conditions.

"T1 plants" refers to plants obtained directly by transforming the plant cell with a genic construct according to the invention and "T2 plants" relates to plants obtained by selfings of T1 plants.

For the purposes of the present invention, "plant of interest" refers to a particular plant on which the present invention is applied and "genetically related species" is used to indicate a plant different from the "plant of interest", which belongs to the same genus. According to the first object of the present invention, there is provided a genic construct, which is capable of inducing recoverable male sterility in a plant of interest.

In particular, said genic construct, according to the above definitions, comprises a blocking construct and a recovery construct.

For the purposes of the present invention, the blocking construct comprises an artificial micro RNA precursor sequence, which is capable of recognizing and targeting a specific target gene thus blocking its function.

In particular, the artificial microRNA has been designed in correspondence to the polymorphism distinguishing the endogenous TAF genes in the plant of interest from a related plant species belonging to the same genus. Within the present invention, the target gene is represented by a gene involved in pollen development, such as, for instance, TAF10 and TAF13 genes.

A preferred artificial micro-RNA (amiRNA) is the one having the sequence corresponding to SEQ ID n° 34 or SEQ ID n° 35 or any 21 bp amiRNA sequence made according the criteria reported by Ossowski et al. (2008) i.e. amiRNA position 2-12 maximum 1 mismatch, cleavage site (position 10 and 11) no mismatch, and up to four mismatches between position 13 and 21, with no more than two consecutive mismatches.

In a preferred embodiment of the invention, the blocking construct further comprises a promoter of the amiRNA precursor expression it is operably linked thereto.

In the present case, there have been used the pTA29 and the pNTM19 promoters, which specifically drive the expression in the anthers *tapetum* and microspore, respectively.

In particular, the pTA29 and pNTM19 promoter are from *Nicotiana tabacum.*

The person skilled in the art will be able to select the most suitable anther specific promoters according to the host plants, such as, for instance, pCHIA, pCHIB for petunia, pEND1 for pea or pTUB8 for rice.

Within the blocking construct there may also be provided one or more genes or transgenic nucleotidic sequences operably associated to the construct in order to be contained within its closest right and left ends. For instance, said genes of transgenic sequences may encode for proteins, enzymes, metabolites, hormones, antibiotics or toxins.

For the purposes of the present invention, the blocking constructs have been cloned into the pGEM-T Easy® (Promega) vector, while other suitable vectors may be used as well, such as, for instance, pENTR™/SD/D-TOPO (Invitrogen) or pDONOR207 (Invitrogen) or other suitable vectors well known to the skilled person.

As above said, the construct of the invention also comprises a genetic element which is able to restore the blocked function, thus reactivating the normal pollen development following a specific external stimulus.

To this end, the recovery element is put under an inducible promoter, which is able to react to said external stimulus activating the transcription of the nucleotidic sequence operably associated to it.

Within the present invention, the rescue construct comprises an amiRNA-insensitive construct, whose sequence, while encoding for the same target gene (TAF10 or TAF13) thus being capable of producing the same activity, is insensitive to the artificial microRNA comprised within the blocking construct.

For the purposes of the present invention, said amiRNA-insensitive form of the target gene, i.e. TAF10 and TAF13 genes, is from a plant genetically related to the plant of interest.

For instance, in case the present application is applied to *Solanum melongena* as the plant of interest, a genetically related species may be represented by *Solanum lycopersicum* (tomato).

It is understood that any other amiRNA-insensitive form of the target gene could be equally suitable for this invention, comprising artificially mutated sequences in order to result not recognizable by the artificial miRNA. In this case, the modification has to be projected in order to maintain the functionality of the original sequence thus to recover the natural function. This result can be achieved considering the degeneracy of the genetic code, therefore, the last nucleotidic base of a triplet encoding for an aminoacid can be substituted without altering the correct translation, in order to provoke the non-recognition by part of the blocking sequence. The generation of a recovery gene insensitive to the blocking construct is particularly efficient when the blocking strategy is based on a amiRNA-mediated silencing, as even the modification of a single base in the target site of the recovery gene could harm the ability of the amiRNA to recognize and therefore to silence the target. Inducible systems are well known in the literature (Wang et al., 2003) and in general any of these systems can be used in the present invention. Preferably, the inducible system is chosen among the systems mediated by glucocorticoidis (positively regulated by dextametason), the heat shock induced system (Sablowski R.W. and Meyerowitz E.M. 1988) and the ethanol inducible system.

The ethanol inducible system (Roslan et al.,2001 Laufs et al., 2003; Caddick et al, 1998) comprises an inducible pALCA promoter activated by the ALCR protein, which is inactive in cells and which is in turn activated by an external stimulus represented by the ethanol.

The treatment with ethanol, in fact, induces a conformation modification in ALCR enabling it to bind to the pALC promoter, then activating it.

A constitutive promoter within the genic recovery element is operably associated to a gene which synthesizes a protein which, when activated by the external stimulus, is able to bind to the inducible promoter and to activate in turn its transcription. In particular, the constitutive promoter can be chosen among constitutive promoters well known in the literature, such as, for instance, the 35S-CaMV, the ubiquitin promoter, the actin and tubulin promoters. In the present invention, 35S-CaMV (p35S) is the constitutive promoter used, which regulates the production of the ALCR protein, which responds to the action of the ethanol. pALCA regulates the transcription of an alternative form of the target gene involved in the pollen development whose sequence is insensitive to the action of the blocking construct. Nevertheless, the alternative versions of the target genes maintain the functionality producing functional proteins able to restore the pollen development.

In particular, in the present invention, it has been used the ethanol inducible system from *A. nidulans.* According to a further object, the present invention discloses a process for the preparation of a genic construct for inducing recoverable male sterility in plants.

Said process, in particular, comprises the preparation of a blocking construct and of a rescue construct as per the above disclosure, which are further digested with suitable restriction enzymes and, according to an embodiment of the present invention, ligated in order to provide a two-component construct. The two-component construct is then digested with proper enzymes and inserted in the T-DNA site of a binary vector, so that the complete final construct of interest is transferred to the host genome in a single genetic locus.

Each of the steps of the cloning scheme further described is realized using techniques of molecular biology known in the field and which can be adapted by a technician depending on the specific type of host, target sequence, promoter and other genetic elements.

According to a still further embodiment, there is provided a method for the preparation of plants whose male sterility is recoverable.

Said method includes the transformation of a plant cell with the genic construct prepared according to the present invention.

Transformation may occur as per well known methods in the art, such as, for instance, transfection with a suitable host cell, biolistic transformation , laser microbeams, microbeads, PEG-mediated protoplast transformation, microinjection, viral transformation.

In a preferred embodiment of the present invention, transfection with a suitable host cell has been performed.

In particular, the binary vectors comprising the two-component constructs above disclosed have been prepared and have then been transferred into a suitable cell for plant cell infection.

Suitable host cells may be, for instance, bacterial cells such as *Agrobacterium tumefaciens or A. rhizogenes.* Other hosts may be selected by the skilled person according to the transformation methods employed as per well known techniques in the art.

In particular, explants are then put into contact with a culture of the infecting host cells bearing the genic construct, then transferred into a suitable medium for growing under suitable conditions.

As per the recovery of male sterility in plants obtained according to the present invention, wherein an ethanol inducible system has been used, said method includes the treatment of the transformed plants with ethanol. Various ways and protocols for performing such treatments are known, such as spraying, root drenching, ethanol vapour, etc as the person skilled in the art will be able to properly select and adapt according to the specific plant species.

For instance, it the case of *Solanum melongena* transformed according to the present invention, flower buds of the T1 transformed plants may be sprayed with a solution of 10-40%, preferably 20-35% and more preferably of 30% ethanol twice a day for at least 2 consecutive days.

The present invention while being described in more details with reference to a specific example of application in *Solanum melongena,* can be implemented in other plants species, among which those belonging to the Brassicaceae, by the person skilled in the art adapting the experimental conditions to the specific needs.

### Blocking construct preparation

TAF10 and TAF13 cDNA sequences have been isolated from total RNA of anthers from both *Solanum melongena* (SEQ ID n° 26 and 27) and *Solanum lycopersicum* (SEQ ID n° 28 and 29) by using the following primers:

**Oligos for TAF10**

| | | |
|---|---|---|
| SEQ ID N°1 | TAF10 | TATACTCCCACTATTCCTGATG |
| | forward | |
| SEQ ID N°2 | TAF10 | TCATTCCTCTCTTGAAGCAGG |
| | reverse | |

**Oligos for TAF13**

| | | |
|---|---|---|
| SEQ ID N°3 | TAF13 | ATGAACAACTCTTCTGCAGG |
| | forward | |
| SEQ ID N°4 | TAF13 | ACTTCCCCTTCACTGATGTG |
| | reverse | |

The PCR reaction has been performed using the Taq phusion (Finnzymes), a taq polymerase with high fidelity and high processivity (4 kilo-bases/minute) and error rate of 4.4 x 10⁻⁷. For the amplification, 10 ng of cDNA of *Solanum melongena,* shortly referred to with Sm, and *Solanum lycopersicum* have been used. Denaturation takes place at 98°C. 35 cycles of amplification have been made. The PCR products, after having been checked on 1% agarose gel, have been purified with the employment of the Wizard® SV Gel and PCR Clean-Up System kit (Promega).

The PCR products have been cloned in the vector pGEM®-T Easy to be conveniently sequenced. The vector pGEM®-T Easy has been prepared by the Promega supplier by cutting the vector pGEM®-5Zf with EcoR V and adding a terminal thymine to the two 3' ends. This vector allows an easy cloning of the templates if the Taq employed for the production of the fragment of interest adds some "Adenines (A)" to the ends of the fragments. Since the used Taq phusion (Finnzymes) is unable to add the A to the ends of our amplified fragments, these have been added in a subsequent phase through incubation at 72°C for 20' with the Taq polymerase supplied by Promega. The PCR products have been purified with the Wizard® SV Gel and PCR Clean-Up System kit (Promega) and then ligated in pGEM®-T Easy, following the indications provided by Promega. In particular, the reaction has been performed in a final volume of 20 µl and incubated for 2 hours at room temperature.

### Design of amiRNA

Both amiRNA sequences targeting TAF10 and TAF13 have been generated following the algorithm and the protocol found in the following web site: http://wmd.weigelworld.org (Schwab et al., 2006). The artificial micro RNAs which mostly target respectively SmTAF10 and SmTAF13 in *S. melongea* resulted to be:

| | | |
|---|---|---|
| 5'-UUGUGGCAAGUCCUUUCUACU-3' | (SEQ ID n°34) | for TAF13; |
| and | | |
| 5'-AUUAAUCGAACGCUAGGACAU-3' | (SEQ ID n°35) | for TAF10 |

Once selected, the amiRNA sequence needs to be engineered into an endogenous microRNA precursor for expression.

Oligo sequences to amplify the complete amiR-SmTAF10 (SEQ ID N° 5 to 8) and amiR-SmTAF13 (SEQ ID N°9 to 12) sequences were retrieved by using the "Oligo" design tool in the above mentioned website and then used to engineer the artificial microRNA versus TAF10 and TAF13 into the endogenous miR319a precursor by site-directed mutagenesis. As a template for the PCRs, the plasmid pRS300, which contains the miR319a precursor in pBSK (cloned via SmaI site), is needed, and has been provided by Detlef Weigel (www.weigelworld.org). Shortly, each amiR-TAF precursor is generated by overlapping PCR. A first round amplifies fragments (Te1) to (Te3), further described. These are subsequently fused in overlapping PCR (to obtain the fragment Te4). For amiR-SmTAF10, the first reaction foresees the use of the primers Vep_0050AmiRNA_B of SEQ ID n. 14 with 10(ami1) of SEQ ID n. 5 (and the amplified fragment is designated Te1) on the starting template vector pRS300. In the second reaction 10 (ami2) of SEQ ID n. 6 and 10 (ami3) of SEQ ID n. 7 are used on the same template pRS300 plasmid (the product is named Te2). Finally, in the third amplification, pBRS300 is amplified with Vep_0049ami_RNA_A-topo of SEQ ID n. 13 and 10(ami4) of SEQ ID n. 8 (the product is named Te3). For amiR-5'mTAF13 the first reaction foresees the use of the primers Vep_0050AmiRNA_B of SEQ ID n. 14 with 13(ami1) of SEQ ID n. 9 (the amplified fragment is designated Te1); on the starting template vector pRS300. In the second reaction the template is always the pRS300 plasmid, but it is amplified with 13(ami2) of SEQ ID n. 10 and 13(ami3) of SEQ ID n. 11 (the product is named Te2). Finally, in the third amplification, pBRS300 is amplified with Vep_0049ami_RNA_A_topo of SEQ ID n. 13 and 13(ami4) of SEQ ID n. 12 (the product is named Te3).
0.5 µl of the purified PCR products Te1-Te2-Te3 were mixed and used as template in overlapping PCR using the primers Vep_0049_amiRNA_A-topo and Vep_0050amiRNA_B (SEQ ID n° 13 and 14). The final template is named T4. For all the PCR reactions Taq phusion (Finnzymes) has been employed. All the PCR products (Te1, Te2, Te3 and Te4) have been separated on a 1% agarose gel, and extracted with the Wizard® SV Gel and PCR Clean-Up System kit (Promega). The Te4 PCR products have been submitted to an additional incubation at 72°C for 20' with the Taq polymerase supplied by Promega for the addition of the adenine tail as described before. The Te4 PCR product have then been purified with the Wizard® SV Gel and PCR Clean-Up System kit (Promega) and then ligated in pGEM®-T Easy (following the indications provided by Promega) to be conveniently sequenced. In particular the reaction has been performed in a final volume of 20 µl and incubated for 2 hours at room temperature.

**Oligos for amiRNA precursor TAF10**

| | |
|---|---|
| SEQ ID 10(ami1) | gaATTAATCGAACGCTAGGACATtctctcttttgtattcc |
| N°5 | |
| SEQ ID 10(ami2) | gaATGTCCTAGCGTTCGATTAATtcaaagagaatcaatga |
| N°6 | |
| SEQ ID 10(ami3) | gaATATCCTAGCGTTGGATTAATtcacaggtcgtgatatg |
| N°7 | |
| SEQ ID 10(ami4) | gaaTTAATCCAACGCTAGGATATtctacatatatattcct |
| N°8 | |

**Oligos for amiRNA precursor TAF13**

| | |
|---|---|
| SEQ ID 13 (ami1) | gaTTGTGGCAAGTCCTTTCTACTtctctcttttgtattc |
| N°9 | |
| SEQ ID 13(ami2) | gaAGTAGAAAGGACTTGCCACAAtcaaagagaatcaatga |
| N°10 | |
| SEQ ID 13(ami3) | gaAGCAGAAAGGACTAGCCACATtcacaggtcgtgatatg |
| N°11 | |
| SEQ ID 13(ami4) | gaATGTGGCTAGTCCTTTCTGCTtctacatatatattcct |
| N°12 | |
| Oligos for complete amiRNA template | |
| SEQ ID VeP_0049AmiRNA A- topo | CACCCTGCAAGGCGATTAAGTTGGGTAAC |
| N°13 | |
| SEQ ID VeP_0050AmiRNA B | GCGGATAACAATTTCACACAGGAAACAG |
| N°14 | |

### Promoter cloning

"pTA29 promoter" and "pNTM19 promoter" from *Nicotiana tabacum* have been kindly provided by Kim Boutilier (Plant Research International, Wageningen University Research) and include the regulatory sequences to direct the expression in the *tapetum* and in the microspore.

The two promoters (corresponding to SEQ ID N° 30 and 31, respectively) were cloned in pGEM-T easy (Promega) using specific primers adding XbaI/SalI restriction sites at 5' end and NcoI/SalI restriction sites at 3' end (Fig 6).

In particular, the following primers have been used: Oligos for cloning promoter pTA29 in pGEM-T easy plus restriction sites

| | |
|---|---|
| SEQ ID N° 15 AtP_1190 | GTCGACTCTAGACTTTTTGGTTAGCGAATGCA |
| (SalI/XbaI) | |
| SEQ ID N° 16 AtP_1191 | CCATGGTCGACTAGCTAATTTCTTTAAGTAAAAAC |
| (NcoI/SalI) | |

Oligos for cloning promoter pNTM19 in pGEM-T easy plus restriction sites

| | | |
|---|---|---|
| SEQ ID N° 17 | AtP_1192 | GTCGACTCTAGAGCCATGGTTAATCACTAAG |
| | (SalI/XbaI) | |
| SEQ ID N° 18 | AtP_1193 | CCATGGTCGACGGATCGATGTTGGTTATC |
| | (NcoI/SalI) | |

Plasmids pGEM-T_amiR-smTAF10, pGEM-T_amiR-TAF13 have been digested with XhoI (new England Biolabs: NEB) in buffer 2 (10 mM Tris-HCl, 50 mM NaCl, 10 mM MgCl₂, 1 mM dithiothreitolo, pH 7.9) plus 100 µg/ml BSA following the instructions supplied by NEB. The reaction has been incubated at 37°C for 2 hours.

pTA29_pGEM-T, pNTM19_pGEM-T plasmids have been digested with SalI (new England Biolabs: NEB) in buffer 3 (50 mM Tris-HCl, 100mM NaCl, 10 mM MgCl₂, 1 mM dithiothreitolo, pH 7.9) plus 100 µg/ml BSA following the instructions supplied by NEB. The reaction has been incubated at 37°C for 2 hours.

Subsequently, the XhoI linearized plasmids pGEM-T_amiR-smTAF10, pGEM-T_amiR-smTAF13 have been ligated to the SalI fragments containing pTA29 and pNTM19. For each ligation reaction, 10 ng of purified pGEM-T-amiR-smTAF10, pGEM-T_amiR-smTAF13 digested with XhoI, and about 20 ng of the two inserts (pTA29 and pNTM19) have been used. For the reaction, the T4 ligase produced by Promega, incubated at 16°C for 12 hours, have been used (see Fig. 7).

At the end 4 different blocking vectors have been obtained:
pGEM-T_pTA29::amiR-smTAF10
pGEM-T pTA29::amiR-smTAF13
pGEM-T_pNTM19::amiR-smTAF10
pGEM-T_pNTM19::amiR-smTAF13
Rescue vectors preparation

To rescue male fertility, the ethanol inducible system from *Aspergillus nidulans* was used (Flipphi et al., 2000). The system comprises the ALCR transcription factor which recognizes the inducible promoter pAlcA. Only in the presence of ethanol, the ALCR protein can bind the pAlcA promoter and thus activate the expression of the downstream gene. In particular, for the purposes of the present invention, a gene coding for a mRNA not recognized by amiRNA is used, which would still be able to produce a substitute functional protein of the corresponding TAF gene in eggplant.

Tomato TAF10 (SEQ ID n° 28) was amplified using primers SEQ ID N° 19 AND N° 2, Tomato TAF13 (SEQ ID n° 29) was amplified using primers SEQ ID N° 20 and N° 4. The two purified sequences were then cloned in pENTR™/SD/D-TOPO (Invitrogen), generating the TOPO_tomatoTAF10 and TOPO_tomatoTAF13 constructs depicted in Figure 8. The reaction has been prepared in a final volume of 10 µl containing 10 ng of the purified PCR product. The reaction mixture has been then incubated at 25°C for 1 hour and 1 µl have been used for the subsequent electroporation of the cells of *E. coli.*

**Oligos for cloning tomato TAF10 in TOPO vector**

| | | |
|---|---|---|
| SEQ ID N° 19 | TAF10 fW_topo | CACCGGTTGGTGGCAGTTGCTAC |
| SEQ ID N° 2 | TAF10 reverse | TCATTCCTCTCTTGAAGCAGG |

**Oligos for cloning tomato TAF13 in TOPO vector**

| | | |
|---|---|---|
| SEQ ID N° 20 | TAF 13fw_topo | CACCGGAGATGATAGCAATCCAC |
| SEQ ID N° 4 | TAF13 reverse | ACTTCCCCTTCACTGATGTG |

### Preparation of the rescue vectors

The complete rescue vectors PL4_pALCA::tomatoTAF10 and PL4_pALCA::tomatoTAF13, have been prepared through a Gateway recombination of LR type between TOPO_tomatoTAF10, TOPO_tomatoTAF13, obtained according to the previous step, and the PL4 gateway vector with the use of the Gateway® system, well known in the art. The Gateway cassette, reading frame A (Life Technologies, Carlsbad, CA) was inserted into the SmaI site of a pL4 vector (Syngenta Ltd, Jeolotts Hill, UK) between the pAlcA promoter and the terminator of the CaMV 35S, generating a pL4_pALCA-Gateway vector. LR recombination reaction has been set in a final volume of 10 µl. The reaction has been incubated at 25°C for about 3 hours. Subsequently, 1 µl has been used for submitting *E. coli* to electroporation. The final plasmids have been checked, extracted by *E. Coli* (see the rescue vectors shown in Fig 9).

Two rescue vector have been obtained:
PL4_pALCA::tomatoTAF10
PL4_pALCA::tomatoTAF13
Preparation of the two-component construct

Blocking vectors obtained according to the procedure above disclosed have been digested with XbaI and the so obtained blocking fragment has been ligated to the corresponding rescue vector, also linearized with partial digeston with XbaI, giving the following two-component construct, (see also Fig 10):
PL4_pTA29::amiR-smTAF10;pALCA::tomatoTAF10
PL4_pTA29::amiR-smTAF13;pALCA::tomatoTAF13
PL4_pNTM19::amiR-smTAF10;pALCA::tomatoTAF10
PL4_pNTM19::amiR-smTAF13;pALCA::tomatoTAF13
wherein the amiR-SmTAF10 final sequence after digestion with XbaI does correspond to SEQ ID n° 32, and the amiR-SmTAF13 final sequence after digestion with XbaI does correspond to SEQ ID n° 33.

### Preparation of the binary vectors

A binary vector containing the p35S::alcR and pAlcA::GUS sequences were kindly provided by Patrick Laufs (Institut Scientifique De Recherche Agronomique, INRA, France). The backbone of the final vector was obtained digesting 35S::ALCR; pALCA::GUS with HindIII (Roche). The digestion has been carried out in buffer B (1 mM Tris-HCl, 0.01 M NaCl, 0.5 mM MgCl₂, 0.1 mM 2-Mercaptoethanol, pH 8), following the supplier instructions. The reaction has been incubated at 37°C for 2 hours. Band corresponding to the vector without pALCA::GUS sequence has been purified from gel employing the Wizard® SV Gel and PCR Clean-Up System kit (Promega). For the reaction, T4 ligase produced by Promega have been used, incubating at 16°C for 12 hours, thus obtaining the NOB839 construct.

NOB839 was digested with HindIII (Roche) in buffer B (1 mM Tris-HCl, 0.01 M NaCl, 0.5 mM MgCl₂, 0.1 mM 2-Mercaptoethanol, pH 8) following the supplier instructions. The reaction has been incubated at 37°C for 2 hours. Two-components vectors have been partially digested with HindIII (Roche) in buffer B following the instructions supplied by Roche. The reaction has been incubated at 37°C for a time optimized to obtain the maximum amount of the desired band around 10'-15' depending on the construct. For each ligation reaction, 20 ng of NOB839 digested with HindIII and about 30 ng of the four different inserts (two-component constructs) partially digested with HindIII and purified have been incubated at 16°C for 12 hours with T4 ligase (Fig. 11). The final four binary vectors for agrobacterium-mediated transformation (see Fig 12) are:
p35S::ALCR;pTA29::amiR-smTAF10;pALCA::tomatoTAF10
p35S::ALCR;pTA29::amiR-smTAF13;pALCA::tomatoTAF13
p35S::ALCR;pNTM19::amiR-smTAF10;pALCA::tomatoTAF10
p35S::ALCR;pNTM19::amiR-smTAF13;pALCA::tomatoTAF13

### EGGPLANT TRANSFORMATION

All the binary plasmids underwent electroporation with *Agrobacterium tumefaciens* using standard techniques well known in the art.

The procedure for eggplant transformation was essentially as described in Rotino et al., (1990, 1997). Leaves and cotyledon explants from two different cv eggplant cultivars (TAL 1/1 and DR2) were pre-coltured for two days in pre-culture medium (Arpaia et al., 1997). For explant infections, an overnight *Agrobacterium tumefaciens* liquid colture was centrifugated and the pellet resuspended at 0.1 OD600 density in MS basal medium, 2%glucose, 200µM acetosyringone pH 5.5. All the explants were infected by dipping in bacteria suspension for 5 min, blotted dry onto sterile filter paper and placed back in the same plates. After 48h the explants were transferred to selective medium without acetosyringone and supplemented with 30 mg/L kanamycin and 500 mg/L cefotaxime. Shoot differentiation and elongation was achieved by transferring calli with compact green nodules to the same selective medium without NAA. Shoots were rooted and propagated in V3 medium (Chambonnet, 1985) without antibiotics. Kanamycin-resistant transgenic plantlets were confirmed by PCR for the presence of the insert, using the following primers, which amplified a fragment of 470 bp of the NPTII coding region.

Oligos for amplification of the NPT II resistance trait
SEQ ID n°21 VeP_0056 NPTII fw TGCTCCTGCCGAGAAAGTAT
SEQ ID n°22 VeP_0063 NPTII rev AGAACTCGTCAAGAAGGCGATAG

### The biological effect in transgenic eggplants of the rescue constructs p35S::ALCR;pALCA::tomatoTAF; pNTM19::amiR-smTAF and p35S::ALCR;pALCA::tomatoTAF; pTA29::amiR-smTAF

Transgenic eggplants for the above mentioned constructs appear phenotypically normal in their vegetative growth and floral morphology. They are indistinguishable from not transformed eggplants, with exception that the transgenic lines for the pALCA::tomatoTAF;pNTM19::amiR-smTAF and p35S::ALCR; pALCA::tomatoTAF;pTA29::amiR-smTAF constructs show an unusually high number of died flowers on the plant, together with a reduced presence of fruit set from open pollination. The transgenic plants don't display any lack of other functions and are able to set fruits with viable seeds when backcrossed with wild type plants as female parent. Fruits are hardly obtained from the transgenic plants expressing the p35S::ALCR;pALCA::tomatoTAF;pNTM19::amiR-smTAF and p35S::ALCR;pALCA::tomatoTAF;pTA29::amiR-smTAF constructs through open pollination and in many cases no fruits have been collected at all. When obtained, these fruits contain viable seeds, which originate PCR positive progenies. Even after several attempts it is almost impossible to use pollen from these plants to backcross eggplant.

### Evaluation of pollen fertility

Evaluation of pollen viability of the transgenic plants transferred to greenhouse and expressing the p35S::ALCR;pALCA::tomatoTAF;pNTMl9::amiR-smTAF and p35S::ALCR;pALCA::tomatoTAF;pTA29::amiR-smTAF constructs have been performed through pollen staining with acetic carmine and evaluation under light microscopy. In most part of the transgenic lines, the pollen does not differ from wild type. In some extreme cases, more than half of pollen is aborted.

A pollen *in vitro* germination assay, serving as reliable measure of pollen fertility, was performed using a PEG 800 culture medium as described in Touarev and Heberle-Bors (2001) (see Table 1 below).

| Chemicals | mg/L | molarity |
|---|---|---|
| CaCl₂ | 111 | 1.0 mM |
| KCl | 75 | 1.0 mM |
| MgSO₄-7H₂O | 200 | 0.8 mM |
| H₃BO₃ | 100 | 1.6 mM |
| CuSO₄ | trace | 30 µM |
| Casein | | |
| hydrolisate | 300 | - |
| PEG 800 | 125000 | - |
| sucrose | 50000 | 146 mM |
| MES | 2900 | 15 mM |
| KOH | pH to 5.9 | |
| Autoclave | | |

Pollen was incubated overnight in PEG 800 medium (dark conditions, 20°C) and evaluation of male fertility was determined on the percentage of germinating pollen grains.

### Pollen germination in transgenic

p35S::ALCR;pALCA::tomatoTAF;pNTM19::amiR-smTAF and p35S::ALCR;pALCA::tomatoTAF;pTA29::amiR-smTAF

Eggplants was tested for several times during the season and, when possible, during the following season. Data are reported in Table 2 below.

Table 2: pollen germination data obtained from all the PCR positive plants belonging to the independent transformed lines bearing the four constructs and the wild type plants

| | | | | | | | | | |
|---|---|---|---|---|---|---|---|---|---|
| construct | N° of independent lines | | Germination ratio (%) | | | | | | |
| | | | | 40 | 30 | 20 | | | |
| | | | >4 | | | | 10 | 5- | 2- |
| | | | | - | - | - | | | |
| | | | 0 | | | | -5 | 2 | 0 |
| | | | | 30 | 20 | 10 | | | |
| P35s::ALCR; | 14 | dr2 | 1 | 3 | 4 | 1 | 2 | 2 | 1 |
| pTA29::amiR- | 8 | tal 1/1 | 0 | 1 | 0 | 0 | 4 | 1 | 2 |
| smTAF10; | | | | | | | | | |
| pALCA::TAF10 | | | | | | | | | |

| | | | | | | | | | |
|---|---|---|---|---|---|---|---|---|---|
| P35S::ALCR; | 18 | dr2 | 1 | 2 | 1 | 7 | 2 | 4 | 1 |
| pNTM19::amiR- | 3 | tal 1/1 | 1 | 0 | 0 | 0 | 1 | 0 | 1 |
| smTAF10; | | | | | | | | | |
| pALCA::TAF10 | | | | | | | | | |
| P35S::ALCR; | 17 | dr2 | 1 | 3 | 2 | 1 | 4 | 3 | 3 |
| pTA29::amiR- | 2 | tal 1/1 | 0 | 0 | 0 | 0 | 0 | 0 | 2 |
| smTAF13; | | | | | | | | | |
| pALCA::TAF13 | | | | | | | | | |
| P35S::ALCR; | 15 | dr2 | 0 | 1 | 1 | 2 | 3 | 6 | 2 |
| pNTM19::amiR- | | | | | | | | | |
| smTAF13; | | | | | | | | | |
| pALCA::TAF13 | | | | | | | | | |
| DR2 wild type | | | 1 | 1 | 0 | 0 | 0 | 0 | 0 |
| TAL 1/1 wild | | | 1 | 1 | 0 | 0 | 0 | 0 | 0 |
| type | | | | | | | | | |

Both *tapetum* and microspore-specific constructs resulted in significantly decreased pollen viability and fertility in the transformed lines. Offsprings (T2) (obtained by selfing) of the best male sterile transgenic lines for each construct were grown in the greenhouse, checked by PCR for the presence of the inserts and evaluated for pollen germination. In each of the considered plants, data of germination ratio do not differ from those of the parental male sterile line they derived from (see Table 3 below).

Table 3: pollen germination data obtained from the F2 progenies of the best male sterile lines for each of the four constructs and the wild type plants

| | | | | | | | | |
|---|---|---|---|---|---|---|---|---|
| | T2 from the best male sterile lines | Germination ratio (%) | | | | | | |
| | | | | | | | | |
| Construct | | | 40- | 30- | 20- | | | |
| | | >40 | | | | 10-5 | 5-2 | 2-0 |
| | | | 30 | 20 | 10 | | | |
| | | | | | | | | |
| p35S::ALCR; | 2 DR2 | 0 | 0 | 0 | 0 | 0 | 1 | 1 |
| pTA29::amiR- | | | | | | | | |
| | 2 TAL | | | | | | | |
| smTAF10; | | 0 | 0 | 0 | 0 | 0 | 0 | 2 |
| | 1/1 | | | | | | | |
| pALCA::TAF10 | | | | | | | | |
| P35S::ALCR; | 2 DR2 | 0 | 0 | 0 | 0 | 0 | 1 | 1 |
| pNTM19::amiR- | | | | | | | | |
| | 1 TAL | | | | | | | |
| smTAF10; | | 0 | 0 | 0 | 0 | 0 | 0 | 1 |
| | 1/1 | | | | | | | |
| pALCA::TAF10 | | | | | | | | |
| P35S::ALCR; | | | | | | | | |
| pTA29::amiR- | | | | | | | | |
| | 2 DR2 | 0 | 0 | 0 | 0 | 0 | 0 | 2 |
| smTAF13; | | | | | | | | |
| pALCA::TAF13 | | | | | | | | |
| P35S::ALCR; | | | | | | | | |
| pNTM19::amiR- | 2 DR2 | 0 | 0 | 0 | 0 | 0 | 0 | 2 |
| smTAF13; | | | | | | | | |

| | | | | | | | | |
|---|---|---|---|---|---|---|---|---|
| pALCA::TAF13 | | | | | | | | |
| DR2 wt | | 1 | 0 | 0 | 0 | 0 | 0 | 0 |
| TAL 1/1 wt | | 1 | 0 | 0 | 0 | 0 | 0 | 0 |

Flowers from the T2 plants have been used as male parents in crossing with wild type plants, but despite the great number of attempts no fruits were obtained, thus demonstrating the high levels of male sterility of the plants.

### Rescue system

The recovery genetic element contains an ethanol inducible promoter which drives the expression of a TAF version from tomato which is insensitive to the action of the amiRNA. Male sterile transgenic lines for each P35S::ALCR;pTA29::amiR-smTAF;pALCA::TAF and P35S::ALCR; pNTM19::amiR-smTAF;pALCA::TAF constructs were subjected to the ethanol-induced recovery of male fertility. Flower spraying with a 30% ethanol solution twice a day for 2 consecutive days is proposed as the most effective to restore normal pollen germination. Flowers at different developing stages from several copies of the selected male sterile lines were sprayed and samples were analyzed for pollen germinations. A recovery of the pollen germination rate in the ethanol sprayed flowers from all the transformed lines was observed (see, for instance Figure 13). Pollen from ethanol-treated flowers was employed for selfing and backcrossed to wild type eggplant and it was able to set fruits, thus confirming that ethanol-rescued pollen is able to perform fertilization and originate viable seeds.

### RNA analysis

Total RNA was collected from whole anthers of the wild type genotypes DR2 and TAL 1/1 and from the best male-sterile transgenic plants (less than 2% of pollen germination ratio) both before and after ethanol induction (24 and 48 hours). Anthers were harvested, frozen in liquid nitrogen and mRNA was extracted and purified according to well established methods.

The complementary cDNA was synthesized by using the following primers:

**Oligos for real time TAF 10**

| | | |
|---|---|---|
| | | |
| SEQ ID n°1 | Taf10 forward | TATACTCCCACTATTCCTGATG |
| SEQ ID n°23 | PrnTaf10 rev2 | TGTCAAGGTAAGGCGTTTGTC |

**Oligos for real time TAF 13**

| | | |
|---|---|---|
| SEQ ID n°24 | PrnTaf13 for2 | GGAGATGATAGCAATCCACTTCC |
| SEQ ID n°25 | PrnTaf13 rev2 | AAGCCTTCCGAGCTTGTTTCAG |

Real time analysis for the expression of the target TAF10 and TAF13 genes revealed an anther-specific reduction in the target TAF expression for transgenic plants expressing the four constructs, while the expressions of the TAF gene in non target tissues or of other non-target TAF were unchanged.

Real time analysis for the expression of the target TAF10 and TAF13 after ethanol treatment revealed a strong and progressive raising (from 24h to 48h treatments) of TAF expression in the transformed male sterile plants, while no difference (or, even, a slight lowering of expression) was observed in TAF expression in ethanol treated wild type flowers (see Figures 14 and 15) The increasing in TAF expression after ethanol induction, together with the complete recovery of the germination rate in flower sprayed with ethanol validate the recovery system utilized.
1. Arpaia S., Mennella G., Onofaro V., Perri E., Sunseri F. e Rotino G.L. (1997). Production of transgenic eggplant (Solanum melongena L.) resistant to Colorado potato beetle (Leptinotarsa decemlineata Say). Theor. Appl. Genet. 95: 329-334
2. Bayer M. and Hess D. (2005) Restoring full pollen fertility in transgenic male-sterile tobacco (Nicotiana tabacum L.) by Cre-mediated site-specific recombination. Molecular breeding 15:193-203
3. Caddick M.X., Greenland A.J., Jepson I. Krause K.P., Qui N., Riddell K.V., Salter M.G., Schuch W., Sonnewald U., And Tomsett B (1998). An ethanol inducible gene switch for plants used to manipulate carbon metabolism. Nature biotechnology, 16: 177-180
4. Chambonnet D. (1985). Culture d'anthères in vitro chez trois Solanacées maraichères: Le piment (Capsicum annuum L.), l'aubergine (Solanum melongena L.), la tomate (Lycopersicum esculentum L.) et obtention de plantes haploides. Thèse de Docteur d'Université. Academie de Montpellier
5. Cho H.J, Shinje K., Minwoo K., and Byung-Dong K. (2001) Production of Transgenic Male Sterile Tobacco Plants with the cDNA encoding a ribosome inactivating protein in Dianthus sinensis L. Mol.Cells 11(3): 326-333
6. Custers J.B.M., Oldenhof T.M., Schrauwen J.A.M., Cordewener J. H.G., Wullems G.J. and van Lookeren Campagne M.M. (1997) Analysis of microspore-specific promoters in transgenic tobacco. Plant Molecular Biology 35: 689-699,
7. Daniell H. (2002) Molecular strategies for gene containment in transgenic crops. Nature 20 :581-587
8. Daniell H., Datta R., Varma S., Gray S., Lee S.B. (1998) Containment of herbicide resistance through genetic engineering of the chloroplast genome. Nature Biotech. 16: 345-348.
9. Derksen J., van Wezel R., Knuiman B. , Ylstra B. and Arjen J. van Tunen (1999) Pollen tubes of flavonol-deficient Petunia show striking alterations in wall structure leading to tube disruption. Planta, 207: 575-581
10. Fisher R., Budde I., and Hain R. 1997. Stilbene synthase gene expression causes changes in flower color and male sterility in tobacco. Plant journal 11(3):489-498
11. Flipphi M, Kocialkowska J, Felenbok B. 2002. Characteristics of physiological inducers of the ethanol utilization (alc) pathway in Aspergillus nidulans. Biochem J 364: 25-31
12. Furumoto T., Tamada Y., Izumida A., Nakatani H., Hata S. and Izui K., (2005) Abundant expression in vascular tissue of plant TAF10, an orthologous gene for TATA box-binding protein-associated factor 10, in Flaveria trinervia and abnormal morphology of Arabidopsis thaliana transformants on its over expression. Plant Cell Physiol. 46(1): 108-117
13. Gao X., Ren F. and Lu Y.T. (2006) The Arabidopsis Mutant stg1 Identifies a Function for TBP-Associated Factor 10 in Plant Osmotic Stress Adaptation. Plant Cell Physiol. 47(9): 1285-1294
14. Kaul, M.L.H. Male sterility in higher plants. Monographs on theor and appl.genet, 10 (Springer, Berlin,1988)
15. Kiete G., Niehaus K., Perlick A.M., Puhler A., and Broer I. (1996) Male sterility in transgenic tobacco plants induced by tapetum-specific deacetylation of the externally applied non-toxic compound N-acetyl-L-phosphinotricin. The plant journal, 9(6): 809-818
16. Koltunow A M., Truettner J., COX K. Wallroth M. and Goldberg R.B. (1990) Different Temporal and Spatial Gene Expression Patterns Occur during Anther Development. The Plant Cell 2: 1201 -1224
17. Kuvshinov Viktor, Koivu Kimmo, Kanerva Anne, Pehu Eija (2001) Molecular control of transgene escape from genetically modified plants. Plant Science 160: 517-522
18. Lago C., Clerici E., Mizzi L., Colombo L., Kater M. (2004) TBP-associated factors in Arabidopsis. Gene 342:231-241
19. Mariani C., De Beuckeleer M., Truettner J., Leemans J., Goldberg R.B.(1990). Induction of male sterility in plants by a chimaeric ribonuclease gene. Nature 347: 737-741
20. Mariani C., Gossele V., De Beuckeleer M., De Block M., Goldberg R.B., De Greef W. & Leemans J. (1992) A chimaeric ribonuclease-inhibitor gene restores fertility to male sterile plants. Nature 357:384-388
21. Napoli C.A. Lemieux C. and Jorgensen R. 1990. Introduction of a chimeric chalcone synthase gene into Petunia results in reversible co-suppression of homologous genes in trans. Plant cell 2:279-289
22. Perez-Prat E. and Van Lookeren Campagne M.M (2002) Hybrid seed production and the challenge of propagating male sterile plants TRENDS in Plant Science Vol.7(5): 199-203
23. Ribarits A., Mamun A.N.K., Li S., Resch T., Fiers M., Heberle-Bors E., Liu C.M. and Touraev A. (2007) Combination of reversible male sterility and doubled haploid production by targeted inactivation of cytoplasmic glutamine synthetase in developing anthers and pollen. Plant Biotechnology Journal 5(4): 483-494
24. Rommens C.M. (2004) All-native DNA transformation: a new approach to plant genetic engineering. Trends in Plant Science 9(9): 457-464
25. Rotino G.L and Gleddie S. (1990) Transformation of eggplant (Solanum melongena L.) using a binary Agrobacterium tumefaciens vector. Plant cell report, 9: 26-29
26. Rotino G.L., Perri E., Zottini M., Sommer H. e Spena A. (1997). Genetic engeenering of partenocarpic plants. Nat. Biotech. 15: 1398-1401
27. Schwab Rebecca, Ossowski Stephan, Riester Markus, Warthmann Norman, and Weigel Detlef (2006) Highly Specific Gene Silencing by Artificial MicroRNAs in Arabidopsis. The Plant Cell, Vol. 18, 1121-1133
28. Touarev A. and Heberle-Bors E.. Methods in molecular Biology, vol 111: plant cell culture protocols pp 281-289.
29. Laufs P., Coen E., Kronenberg J., Traas J., and Doonan J. (2003) separable roles of UFO during floral development revealed by conditional restoration of gene function. Development 130:785-796.
30. Roslan, H.A., Salter M.G., Wood C.D., White M. R., Croft K.P., Robson F., Coupland G., Doonan J., Laufs P., Tomsett A.B., and Caddick M.X.(2001) Characterization of the ethanol inducible alc gene-expression system in Arabidopsis Thaliana. Polant J.28: 225-235.
31. Sablowski R.W. and Meyerowitz E.M. (1998) Temperature-sensitive splicing in the floral homeotic mutant apetala3-1. Plant cell.10:1453-1463.
32. Wang R., Zhou X., and wang X (2003). Chemically regulated expression systems and their applications in transgenic plants. Transgenic Research, 12:529-540

## Claims

1. A genic construct for inducing recoverale male sterility in a plant of interest, comprising:
- a blocking genic construct comprising a anther-specific promoter operably associated to a nucleotidic sequence capable of inactivating a target gene involved in pollen development;
- a recovery genic construct comprising an inducible promoter operably associated to an insensitive form of said gene involved in pollen development.

2. The genic construct of claim 1, further comprising one or more genes or transgenic nucleotidic sequences operably associated to the construct in order to be contained within its closed right and left ends.

3. The genic construct of claim 2, wherein said genes or transgenic nucleotidic sequences may be genic elements which encode for a protein, an enzyme, metabolites, hormones, antibiotics or toxins.

4. The genic construct according to any one of claims from 1 to 3, wherein said sequence capable of inactivating a gene involved in pollen development encodes for a nucleotidic sequence capable of binding itself to an target site of said gene involved in the pollen development to inactivate it.

5. The genic construct according to any one of claims from 1 to 4, wherein said sequence capable of inactivating a gene involved in pollen development hybridizes with a target sequence to be inactivated and is able to provoke a breakage inside it or preventing the translation of mRNA.

6. The genic construct according to claim 5, wherein said sequence capable of inactivating said target gene within the blocking construct is a sequence encoding for the expression of an artificial micro RNA or is an interference RNA.

7. The genic construct according to claims 1 to 6, wherein said target gene encodes for a basal transcription factor involved in the pollen development in plants.

8. The genic construct according to claim 7, wherein said basal transcription factor involved in pollen development in plants belongs to the family of TAF genes.

9. The genic construct according to claim 8, wherein said basal transcription factor sequence is TAF10 gene or TAF13 gene.

10. The genic construct according to claims 1 to 9, wherein said anther-specific promoter is selected in the group comprising pTA29 and pNTM19.

11. The genic construct according to claim 8 or 9, wherein said plant of interest is *Solanum melongena.*

12. The genic construct according to any one of the claims from 1 to 11, wherein said nucleotidic sequence capable of inactivating a gene involved in the pollen development comprises the sequence of SEQ ID n°34 or 35.

13. A genic construct comprising the sequence corresponding to SEQ ID n°32 or 33.

14. The genic construct according to any one of the claims from 1 to 13, wherein the inducible promoter within the recovery genic construct is capable to react to an external stimulus by activating the transcription of the sequence of the gene involved in the pollen development operably associated thereto.

15. The genic construct according to claim 14, wherein said inducible promoter is chosen among the promoters of the inducible system mediated by glucocorticoids, of the inducible system mediated by heat shock and of the ethanol inducible system and preferably is the ethanol inducible system.

16. The genic construct according to any one of the claims from 1 to 15, further comprising a costitutive promoter operably associated to the gene encoding for a protein which, when activated by the external stimulus, is able to bind to the inducible promoter and to activate its transcription.

17. The genic construct according to claim 16, wherein said constitutive promoter is selected in the group comprising the 35S-CaMV promoter, the ubiquitin promoter, the actin promoter and the tubulin promoter and preferably is the 35S-CaMV promoter.

18. The genic construct according to any one of claims from 1 to 17, wherein the sequence of the gene involved in the pollen development, which is insensitive to the nucleotidic sequence capable of inactivating the gene involved in the pollen development within the blocking genic construct, is represented by a form of the same gene which has been isolated from a species genetically related to the plant of interest, wherein genetically related means that it belongs to the same genus of the plant of interest.

19. The genic construct according to claim 18, wherein the genetically related species to the plant of interest is *Solanum lycopersicum.*

20. The genic construct according to claim 18, wherein said sequence of the gene involved in the pollen development, which is insensitive to the nucleotidic sequence capable of inactivating the gene involved in the pollen development within the blocking genic construct, is TAF10 or TAF13 from *Solanum lycopersicum.*

21. The genic construct according to any one of claims from 1 to 20, wherein such form of the gene which results insensitive to the inactivation is represented by an artificially mutated form of the sequence which is active in its natural function but whose nucleotidic composition is different from the native form.

22. A method for the preparation of a genic construct for the induction of recoverable male sterility in a plant of interest, according to any one of claims from 1 to 21, comprising the steps of:
1) isolation from the genome of the species of interest of the nucleic sequence of a plant pollen-specific promoter;
2) amplification of said sequence;
3) digestion of the sequence with appropriate restriction enzymes to allow the insertion into a suitable cloning plasmid;
4) digestion of a suitable cloning plasmid with the same restriction enzymes used in step 3) to consent the integration of said sequence in said plasmid;
5) integration of said pollen-specific promoter in said plasmid;
6) isolation from the genome of interest of a sequence of a target gene involved in pollen development;
7) amplification of said sequence;
8) synthesis of a sequence capable of hybridizing and inactivating said target sequence;
9) cloning said sequence capable of hybridizing and inactivating the target sequence in appropriate plasmids;
10) recombining the products of step 5) and of step 9) to obtain a genic construct for the recoverable inhibition of pollen fertility in plant;
11) isolating from the genome of a species genetically related to the species of interest a gene involved in the pollen development which is insensitive to the inactivating sequence of step 8) or, alternatively, making available an artificially mutated form of the sequence which is active in its natural function but whose nucleotidic composition is different from the native form;
12) recombining the product of step 11) in an appropriate vector;
13) isolating from the genome of interest the nucleotidic sequence of an inducible promoter;
14) amplificating the sequence of step 13)
15) digesting the sequence of step 14) with appropriate restriction enzymes to consent its insertion into a suitable cloning plasmid;
16) digesting a suitable cloning plasmid with the same restriction enzymes used in the preceding step to consent the integration of said sequence in said plasmid;
17) integrating the product of step 15) in the plasmid of step 16);
18) ligating the insert of the vector obtained in step 12) and the linearized plasmid of step 17) to obtain a plasmid containing the genic construct for inducing recoverably male sterility;
19) recombination between the products of step 10) and of step 18) to obtain the genic construct for the recoverable inhibition of the development of pollen.

23. The method for the preparation of a genic construct for the induction of recoverable male sterility in a plant of interest according to claim 22, wherein the target gene of step 6) is represented by TAF10 or TAF13.

24. The method for the preparation of a genic construct for the induction of recoverable male sterility in a plant of interest according to claim 22 or 23, wherein the plant of interest is represented by *Solanum melongena.*

25. The method for the preparation of a genic construct for the induction of recoverable male sterility in a plant of interest according to claim 24, wherein the species genetically related to the species of interest is *S. lycopersicum.*

26. The method for the transformation of a plant through the genic construct according to any one of claims from 1 to 21, comprising the steps of:
a) making available a genic construct for the recoverable inhibition of the pollen development in transgenic plants according to any one of the claims from 1 to 21;
b) inserting said construct in a host organism capable to transform a target organism or parts of this one
c) transforming said plant organism or parts of it with said host organism bearing said construct.

27. The method according to claim 26, wherein step a) comprises the cloning and the transferring of said genic construct in heterologous expression vectors, such as binary vectors.

28. The method according to claim 26 or 27, wherein step b) comprises the introduction of cloning plasmids containing the genic construct of any one of claims 1 to 21 by electroporation in a host organism.

29. The method according to claim 26 to 28, wherein the host organism of step b) is selected among *A*. *tumefaciens* or *A*. *rhizogenes.*

30. The methods according to claim 26 to 29, wherein step c) is performed by biolistic transformation, microbeads, PEG-mediated protoplast transformation, microinjection, viral transfection or protoplast electroporation through the genic construct according to any one of claims from 1 to 21.

31. Seeds, plants or parts of genetically modified plants comprising the genic construct according to any one of claims from 1 to 21.
